(19) Europäisches Patentamt

European Patent Office

Office européen des brevets

(11) Veröffentlichungsnummer: **0 307 646 B1**

(12) **EUROPÄISCHE PATENTSCHRIFT**

(45) Veröffentlichungstag der Patentschrift: **20.05.92**

(21) Anmeldenummer: **88113469.6**

(22) Anmeldetag: **19.08.88**

(51) Int. Cl.⁵: **A61F 2/32**, A61F 2/36

(54) **Femur-Hüftgelenk-Endoprothese.**

(30) Priorität: **18.09.87 DE 3731421**

(43) Veröffentlichungstag der Anmeldung:
**22.03.89 Patentblatt 89/12**

(45) Bekanntmachung des Hinweises auf die
Patenterteilung:
**20.05.92 Patentblatt 92/21**

(84) Benannte Vertragsstaaten:
**AT BE CH DE ES FR GB GR IT LI LU NL SE**

(56) Entgegenhaltungen:
**EP-A- 0 124 443**
**US-A- 4 657 551**
**US-E- 32 471**

(73) Patentinhaber: **S + G IMPLANTS GMBH**
**Grapengiesserstrasse 34**
**W-2400 Lübeck(DE)**

(72) Erfinder: **Grundei, Hans**
**Hamburger Strasse 89**
**W-2400 Lübeck(DE)**

(74) Vertreter: **Eisenführ, Speiser & Strasse**
**Martinistrasse 24**
**W-2800 Bremen 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung geht aus von einer Femur-Hüftgelenk-Endoprothese nach der DE-PS 2 851 598, bei der der vom Gelenkkopf konisch auslaufende Stiel auf seiner Länge in einer Wellenlinie kleiner Amplitude im oberen Schaftbereich mit Halbwelle entgegengesetzt zur Gehrichtung konkav nach hinten und daran anschließend mit unterer Halbwelle in Gehrichtung konkav nach vorn gekrümmt ist.

Durch die vorerwähnte Ausbildung der Endoprothese ist das Einsetzen des Stieles mit dem Gelenkkopf zwangsmäßig nur in einer Lage möglich.

In der Praxis ändert sich nun bei gleichem oder etwa gleichbleibendem Gelenkkopf der erforderliche Durchmesser und auch die Länge des Stieles für die verschiedenen Patienten, so daß die Klinik für alle vorkommenden Fälle viele Femur-Hüftgelenk-Endoprothesen vorrätig halten muß, was eine erhebliche Kostenbelastung bedeutet.

Die Aufgabe der Erfindung besteht daher darin, unter Verwendung von Femur-Hüftgelenk-Endoprothesen der eingangs erwähnten Art, die Lagerhaltung solcher Endoprothesen und die Kosten hierzu so klein wie möglich zu machen.

Diese Aufgabe wird durch die kennzeichnenden Merkmale des Anspruches 1 gelöst.

Durch diese Lösung braucht eine Klinik praktisch nur viele im Durchmesser und in der Länge verschiedene Stiele,aber nur einige wenige gleiche Gelenkköpfe auf Lager zu halten, was die Kosten für die Klinik wesentlich herabsetzt, da die Kosten für die Lagerhaltung vieler Stiele und nur weniger gleicher Gelenkköpfe sehr viel günstiger ist als die Lagerhaltung einteiliger Femur-Hüftgelenk-Endoprothesen.

Die Erfindung wird nachstehend anhand der Zeichnung erläutert: in der ein Ausführungsbeispiel dargestellt ist. Es zeigt:

Figur 1    eine Vorderansicht der Hüftgelenk-Endoprothese nach der Erfindung mit teilweisem Längsschnitt,

Figur 2    eine Seitenansicht zu Figur 1,

Figur 3    den Stiel nach Figur 2 in der gleichen Lage ohne Gelenkkopf.

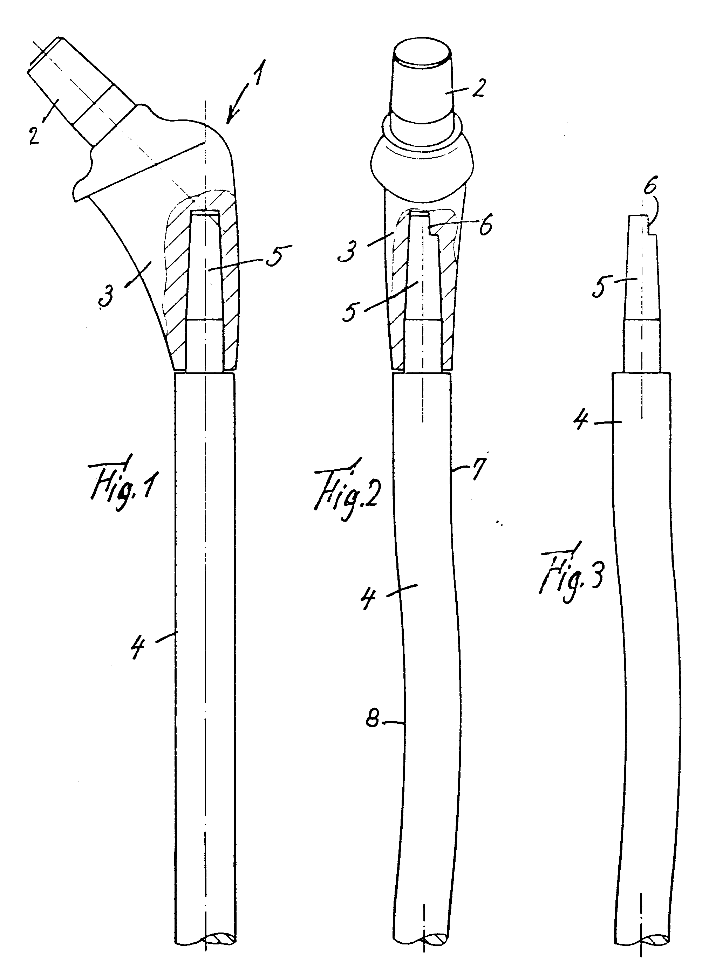

Die Femur-Hüftgelenk-Endoprothese besteht aus einem Kopfteil 1, deren Hals 2 eine nicht dargestellte Gelenkkugel trägt. Der Kopfteil 1 bildet mit dem einen oberen kurzen Stiel 3 ein vom Stiel 4 getrenntes Teil. Das Oberende des Stieles 4 ist mit einem Konus 5 versehen, der am Ende mit einer Abflachung 6 ver sehen ist. Unter dem Ausdruck "Abflachung" wird eine vom Konusumfang zum Ende verlaufende ebene Schnittfläche oder auch eine abgestufte Fläche entsprechend Figur 2 und 3 verstanden.

Dadurch, daß der Stiel 4 auf seiner Länge in einer Wellenlinie kleiner Amplitude ausgebildet ist, wie eingangs erwähnt ist, nimmt er im Markraum des Femurs nur eine bestimmte feste Lage ein, wobei die Konkavität 7 im oberen Bereich nach hinten gerichtet ist und die Konkavität im unteren Halbwellenbereich bei 8 nach vorn gerichtet ist. Wenn nun der Konus 5 die Abflachung 6 und die Konusausnehmung im Kopfteil I einen der Abflachung entsprechenden Vorsprung besitzt, so ist damit neben den selbsthemmenden Eingriff des Konus 5 in die Konusausnehmung des Kopfes (1) zusätzlich ein Formschluß erreicht, durch den der Kopfteil 1 nur eine einzige Lage gegenüber dem Stiel 4 einnehmen kann.

Durch die zweiteilige Ausbildung der Femur-Hüftgelenk-Endoprothese und die kraft-formschlüssige Verbindung der beiden Gelenkteile 3 und 4 ist es auf einfache Weise möglich geworden, daß die einleitend gestellte Aufgabe auf einfache Weise gelöst ist.

**Patentansprüche**

1.    Femur-Hüftgelenk-Endoprothese mit einem vom Gelenkkopf (1) konisch auslaufenden Stiel (4), der auf seiner Länge in einer Wellenlinie kleiner Amplitude im oberen Schaftbereich mit Halbwelle entgegengesetzt zur Gehrichtung konkav nach hinten und daran anschließend mit unterer Halbwelle in Gehrichtung konkav nach vorn gekrümmt ist, dadurch gekennzeichnet, daß der Gelenkkopf (1) mit kurzem oberen Stielteil (3) ein vom Stiel (4) getrenntes Teil ist und daß der Stiel (4) mit einem Konus (5) selbsthemmend und formschlüssig in eine Konusausnehmung des Kopfes eingreift.

2.    Endoprothese nach Anpsruch 1, dadurch gekennzwichnet, daß der formschlüssige Eingriff aus einer Abflachung (6) des Konusoberendes (5) und einem der Abflachung angepaßten Vorsprung der Konusausnehmung des Gelenkkopfes (1) besteht.

**Claims**

1.    A femur-hip joint endoprosthesis having a shaft (4) which tapers from the head (1), and, over its length in a wave line of small amplitude, half wave of the shaft is curved concavely backwards in the upper shaft region opposite to the direction of walking and subsequent thereto the lower half wave is curved concavely forwards in the direction of walking, **characterised in that** the head (1) including a short upper shaft section (3) is a part separated from the shaft (4)

**and in that** the shaft (4) inlcuding a cone (5) engages in a conical recess of the head in a self-locking and positive manner.

2. An endoprosthesis according to Claim 1, **characterised in that** the positive engagement consists of a flattened part (6) of the upper end of the cone (5) and a projection of the conical recess of the head (1) adapted to the flattened part.

**Revendications**

1. Endoprothèse pour l'articulation fémur-hanche comprenant une tige (4) qui part sous une forme conique depuis une tête d'articulation (1) et qui, sur sa longueur, est courbée suivant une ligne ondulée de petite amplitude, dans la zone de tige supérieure de manière concave vers l'arrière avec une demi-onde en sens opposé au sens de la marche et, à la suite de cela, de manière concave vers l'avant avec une demi-onde inférieure dans le sens de la marche, caractérisée en ce que la tête d'articulation (1) est, avec une courte partie de tige supérieure (3), une partie séparée de la tige (4) et en ce que la tige (4) présentant un cône (5) pénètre, d'une manière autoblocante et en réalisant une liaison par la forme, dans un évidement conique de la tête.

2. Endoprothèse suivant la revendication 1, caractérisée en ce que l'engagement réalisant une liaison par la forme est constitué d'un aplatissement (6) de l'extrémité supérieure du cône (5) et d'une saillie, adaptée à l'aplatissement, de l'évidement conique de la tête d'articulation (1).

*Fig.1*

*Fig.2*

*Fig.3*